# EUROPEAN PATENT SPECIFICATION

(11) **EP 1 647 269 B1**
(45) Date of publication and mention of the grant of the patent: **12.04.2017**
(21) Application number: 04738328.6
(22) Date of filing: 02.07.2004
(51) Int. Cl.: A61K 9/14, A61K 47/26, A61K 47/36, A61K 47/38, A61K 47/40, A61K 31/00, A61K 36/00

(54) **MATRIX ADJUVANTS AND THE DROP PILLS PREPARED WITH THEM**
MATRIX-ADJUVANTIEN UND DIE DAMIT HERGESTELLTEN TROPFEN UND PILLEN
ADJUVANTS DE MATRICE ET GRANULES PREPARÉES A L'AIDE DES DITS ADJUVANTS

(30) Priority: 02.07.2003 CN 03145615; 17.03.2004 CN 200410018761
(43) Date of publication of application: 19.04.2006
(73) Proprietor: Tasly Pharmaceutical Group Co., Ltd., Tianjin 300402 (CN)
(72) Inventor: CHEN, Jianming No. 1 Liaohedong Road, Tianjin 300402 (CN); YAN, Xijun No. 1 Liaohedong Road Xinyibai Avenue, Tianjin 300402 (CN); YANG, Yuewu No. 1 Liaohedong Road Xinyibai Avenue, Tianjin 300402 (CN); LU, Wenliang No. 1 Liaohedong Road, Tianjin 300402 (CN); ZHU, Yonghong No. 1 Liaohedong Road, Tianjin 300402 (CN); YE, Zhengliang No. 1 Liaohedong Road, Tianjin 300402 (CN); WANG, Wei No. 1 Liaohedong Road Xinyibai Avenue, Tianjin 300402 (CN); ZHU, Guoguang No. 1 Liaohedong Road, Tianjin 300402 (CN); ZHENG, Zhigang No. 1 Liaohedong Road, Tianjin 300402 (CN); WANG, Shuangming No. 1 Liaohedong Road, Tianjin 300402 (CN)
(74) Representative: Pfenning, Meinig & Partner mbB
(86) International application number: PCT/CN2004/000730
(87) International publication number: WO 2005/002547

(56) References cited:
- EP-A- 1 388 344
- WO-A-98/42311
- US-A- 4 324 779
- US-A- 5 876 754
- DATABASE WPI Week 200376 Thomson Scientific, London, GB; AN 2003-805041 XP002521858 & CN 1 394 606 A (ZHANG Y) 5 February 2003 (2003-02-05) & CN 1 394 606 A (ZHANG YICHENG [CN]) 5 February 2003 (2003-02-05)
- PHARMACEUTICS, THE PEOPLE'S HYGIENE PUBLICATION vol. 4, April 2004, pages 307 - 308
- CHEN HAO ET AL.: 'Solid dispersion pharmaceutical technique' SHANGDONG ZHONGYI ZAZHI vol. 9, no. 5, March 2000, pages 304 - 305, XP008103838
- PAN HAIXI ET AL.: 'Traditional carriers for solid dispersion' XIBEI JOURNAL OF PHARMACY vol. 12, no. 6, December 1997, pages 277 - 278, XP008103649
- LUAN LIBIAO ET AL.: 'Preparation and in vitro dissolution of ibuprofen pills' CHINESE JOURNAL OF PHARMACEUTICALS vol. 31, no. 9, September 2000, pages 399 - 401, XP008065695
- HOU PENG ET AL.: 'research progress in practical pharmacy of solid dispersion technique' CHINESE JOURNAL OF NEW DRUGS vol. 12, no. 4, April 2003, pages 245 - 249, XP002999229

## Description

### Field of the invention

The present invention relates to pharmaceutical formulation. More specifically, the present invention relates to the matrix adjuvants for drop pills and the drop pills prepared with them.

### Background of the invention

Drop pill is prepared by dripping, namely, the solid or liquid drug is dissolved, suspended or emulsified into the matrix, then the mixture is dropped into the matrix -insoluble coolant to form the pills with the shape of sphere or oblate spheroid by congealing. It can be administered orally or intracavitarily, or can be used to prepare solution, etc. Compared with other pharmaceutical formulations, the drop pill has many advantages such as easy operation of the dropping machine, small weight variation of pills, accurate content of the pharmaceutical active ingredient, low loss of drug in the processing, stability of quality, and capacity to make the drug exhibit high efficacy, enduring efficacy and quick efficacy, etc. The formulation of drop pill is especially suitable for those poorly water-soluble drugs which are difficult to be absorbed and the herbs containing volatile oil as their effective components. The development of drop pill formulation satisfies the basic requirements for the modern pharmaceutical formulation such as "three less", i.e. less amount of administration, less toxic and less side effect; "three efficacy", i.e. high efficacy, long efficacy and quick efficacy, and easiness to be administered, carried and stored, thus it is promising for a huge potential market.

Despite the recent great development in the manufacturing machine, process and variety of medicine for drop pill, the development of research and application on the novel matrix adjuvants for drop pill is still slow. So far, polyethylene glycol (PEG) is commonly used as matrix adjuvant for most of the drop pills, and meanwhile, polyoxyethylene monostearate, glutin, poloxamer, polyether and the like are selected occasionally. In terms of source, polyethylene glycol, polyoxyethylene monostearate, poloxamer and polyether and the like are produced by artificial synthsis. Glutin is derived from natural materials, but it is mainly derived from the skin and bone of animals. In terms of safety, the chemically synthesized materials such as polyethylene glycol, polyoxyethylene monostearate, poloxamer, polyether and the like are pharmaceutically acceptable, but they can cause hemolyzation to some extent. Furthermore, some chemical components such as ethylene oxide, epoxy propane and the like, which have toxic and side effects to human, are inevitably mixed into these materials during the process of chemical synthesis. In addition, these chemically synthesized materials may be incompatible with many drugs such as salicylic acid, diphenhydramine, potassium penicillin G, tetracycline, etc., which reduces the curative effect of these drugs. As for glutin, its application is limited because the original auxiliary materials derived from animals are prohibited in order to avoid the animal diseases such as mad cow disease, foot-and-mouth disease, etc.

Additionally, long-term study showed that the drop pills with the polyethylene glycol were unstable because of various problems, for example, they are prone to aging and cracking, etc. So how to expand the application range of matrix for drop pill and make them suitable for the extracts of Chinese medicines with different properties (hydrophilicity or lipophilicity) to formulating to drop pills, and increase the drug loading of drop pill formulation are urgent problems need to be solved.

Therefore, studying and developing some novel, safe and non-toxic matrix adjuvants of drop pill is significant for improving the product quality of drop pill, widening the application range of drop pill, driving the development of drop pill formulation and promoting the internationalization of the drop pill formulation. However, the demand of process of drop pill for the matrix adjuvants is very strict, the drop pill products which satisfy the requirements of quality are usually difficult to be prepared after the matrix adjuvant is changed. Therefore, no substitutes of polyethylene glycol which are more suitable to be used as matrix adjuvants are available now.

### Summary of the Invention

The objectives of the present invention are to change the situation that the chemically synthesized materials such as polyethylene glycol has long been used as matrix adjuvant which leads to the toxic and side effects and the scarcity of the adjuvant materials derived from animals, to reduce the amount of usage of the chemically synthesized materials and the materials derived from animals, to provide a natural, safe and non-toxic materials derived from plants used as matrix adjuvant or the main components of the matrix adjuvant for drop pills, to promote the development of the drop pills formulation, and to accelerate the internationalization of the drop pill products.

Another objective of the present invention is to provide a process for preparing drop pills using or mainly using the natural matrix adjuvants derived from plants.

Specifically, the present invention relates to the following:
1. A drop pill comprising the pharmaceutical active ingredient and at least one of the pharmaceutically acceptable matrix adjuvants selected from a group consisting of monosaccharide, oligosaccharide, polysaccharide, sugar ester, sugar alcohol, alpha-hydroxy acid, higher fatty acid derivative, higher aliphatic alcohol, polyol, urea, and poly(ethylene oxide) derivative.
2. The drop pill according to 1, wherein said pharmaceutical active ingredient is a extract of crude drug.
3. The drop pill according to 1, wherein said pharmaceutical active ingredient is a chemically synthesized drug, antibiotic or biochemical drug.
4. The drop pill according to any one of 1-3, wherein as the matrix adjuvants, said monosaccharide is D-ribose, fructose, glucose, xylose; said oligosaccharide is trehalose, raffinose, maltose; said polysaccharide is gelose; said sugar ester is sucrose ester, D-ribonic acid-γ-lactone; said sugar alcohol is erythritol, sorbitol, xylitol, arabitol, isomaltitol, lactitol; said alpha-hydroxy acid is malic acid, citric acid; said higher fatty acid derivative is sodium stearate, glycerin stearate, glycerin palmitate, shellac; said higher aliphatic alcohol is cetyl alcohol, stearyl alcohol; said polyol is phenyl ethanediol; said poly(ethylene oxide) derivative is polyoxyethylene monosteatate, polyoxyethylene alkyl ether, and the above-mentioned compounds containing crystal water.
5. The drop pill according to any one of 1-3, wherein said matrix adjuvant is at least one of natural adjuvants derived from plants which are selected from a group consisting of the following: sorbitol, xylitol, lactitol, maltose, sucrose ester, and the above-mentioned compounds containing crystal water.
6. The drop pill according to any one of 1-5, wherein said drop pill further comprises at least one of plastifying components selected from a group consisting of the following: starch and their derivatives, cellulose and their derivatives, arabic gum, dextran, chitin, sesbania gum, carrageen gum, Indian gum, danish agar, tragacanth, carrageenin, tamarind gum, pectin, xanthan gum, alginic acid and the salts thereof, dextrin, cyclodextrin, agar, lactose; polyvinylpyrrolidone, cross-linked polyvinylpyrrolodione, carbomer, polyvinyl alcohol, acrylic acid resin, poloxamer, silicon dioxide, glutin, glycerin monostearate, polyoxyethylene monostearate.
7. The drop pill according to 6, wherein said plastifying components is one or more substances selected from a group consisting of the following: pregelatinized starch, carboxymethyl starch, methyl cellulose, sodium carboxymethyl cellulose, hydroxypropyl methyl cellulose, arabic gum, alginic acid, dextrin, cyclodextrin, agar, lactose, glycerin monostearate, polyoxyethylene monostearate, cross-linked sodium carboxylmethyl cellulose, silicon dioxide.
8. The drop pill according to 6 or 7, wherein said matrix adjuvant includes lactitol and starch.
9. The drop pill according to 6 or 7, wherein said matrix adjuvant includes xylitol and arabic gum.
10. The drop pill according to 6 or 7, wherein said matrix adjuvant includes sucrose ester and glycerin monostearate or polyoxyethylene monostearate.
11. The drop pill according to 6 or 7, wherein said matrix adjuvant includes sucrose ester, polyoxyethylene monostearate and cross-linked sodium carboxylmethyl cellulose.
12. The drop pill according to 6 or 7, wherein said matrix adjuvant includes sucrose ester, polyoxyethylene monostearate, cross-linked sodium carboxylmethyl cellulose and silicon dioxide.
13. The drop pill according to 1, wherein the weight ratio of the matrix adjuvant to the pharmaceutical active ingredient is in the range of 1:0.1∼1:1.
14. The drop pill according to 1, wherein the weight ratio of the matrix adjuvant to the pharmaceutical active ingredient is in the range of 1:0.1∼1:0.6.
15. A matrix adjuvant for drop pill comprising xylitol and starch with the weight ratio of 1:0.2∼1:0.3.
16. A matrix adjuvant for drop pill comprising lactitol and starch with the weight ratio of 1:0.2∼1:0.3.
17. A matrix adjuvant for drop pill comprising xylitol and arabic gum with the weight ratio of 1:0.2∼1:0.4.
18. A matrix adjuvant for drop pill comprising sucrose ester and glycerin monostearate or polyoxyethylene monostearate with the weight ratio of 1:0.1∼1:1.
19. A matrix adjuvant for drop pill comprising sucrose ester, polyoxyethylene monostearate and cross-linked sodium carboxyl methyl cellulose with the weight ratio of 1:(0.1∼1):(0.1∼1).
20. A matrix adjuvant for drop pill comprising sucrose ester, the plastifying components including polyoxyethylene monostearate, cross-linked sodium carboxylmethyl cellulose and silicon dioxide with the weight ratio of 15:(7∼15):(0.1∼2):(0.1∼2).

### Detailed description of the invention

The drop pills of the present invention are composed of drugs and natural matrix adjuvants for drop pill which mainly derived from plants. Said drugs comprise the extract from crude drug(s), the chemically synthesized drug, antibiotic and biochemical drug.

The extract from crude drug(s) of the present invention refers to the extract of all kinds of plants, animals, fungi or minerals and the like which are commonly used as traditional Chinese medicines(TCM) in China or as natural medicines in other countries, including the extract of one or more kinds of crude drugs or mixtures of the extracts. Said extracts, namely the effective fractions of medicinal materials, for example, the extracting solution, extract and liquid extract, etc., which comprise the hydrophilic and lipophilic components, and the components of volatile oil etc. are obtained by those methods commonly used in the art, for example, the crude drugs are dipped, extracted, or decocted by water or organic solvent such as methanol, ethanol, ethyl ether, petroleum ether, acetone, chloroform, etc. without or in the presence of acid or alkali. The above components may be used after further purification by the well-known methods in the art such as ion exchange resin, macroporous absorption resin, ultrafiltration membrane, silicon gel column or alumina column chromatography, high performance liquid chromatography, and the like. The mixtures of exacts above-mentioned may be obtained by extracting the aforehand mixtures of crude drugs or mixing the extracts of crude drugs, or combining the above two methods. Said extracts of crude drugs usually include, but are not limited to, all kinds of well-known natural organic compounds such as sugar, protein, nucleic acid, alkaloid, glucoside, coumarin, and lignin, volatile oil of monoterpenes and diterpenes, triterpenoids and triterpenoid saponins, cardiac glycoside and steroidal saponins, flavones, quinones, polyphenols, etc. Said extracts of the present invention generally do not includes monomer drug used as the chemically cure drugs extracted from plants and animals such as morphine, pacilitaxel, tetrodotoxin, reserpine, berberine, artemisinin, etc. Said extracts of crude drug of the present invention can be prepared by the well-known methods in the art or commercially available.

Said chemically synthesized drugs, antibiotics or biochemical drugs of this invention refer to those which are monomer and small molecule chemically therapeutic drugs, antibiotics, protein, nucleic acids and the like with defined structure and/or unitary characteristic, and obtained in the method of chemical synthesis or extracting from leavening. The monomer drugs extracted from plants and animals are used as monomer drug itself, or further transferred to other substances which can be used as drugs. Said chemically synthesized drugs, antibiotics or biochemical drugs of this invention include, but are not limited to, sedative and hypnotic agents such as barbiturates, diazepines etc.; analgesics such as morphine, pethidine, methadone, etc; anticholinergic drugs such as atropine, anisodamine, etc.; α and β-receptor blockers; histamine H1,H2-recepor antagonists; HMG coenzyme A inhibitor; curing drugs of hepatobiliary diseases such as Schizandrin B, Schizandrin C, bifendate, silymarin, ursodeoxycholic acid, etc; antipyretic analgesics and non-steroidal anti-inflammatory drugs; anti-tumor drugs such as alkylating agents, natural and synthesized alkaloids, taxanes, anticancer antibiotics; natural and semi-synthesized, full synthesized antibiotics such as β-lactams, macrolides, aminoglycosides, chloramphenicols, etc.; synthesized antibacterial drugs such as quinolones, sulfanilamide, etc; antifungal drugs such as azoles, etc; antiviral drugs such as nucleosides or non-nucleosides; antiparasitic drugs; hormones such as prostaglandins, peptides, steroids; vitamins, immune nucleic acid, bacterin, etc. They also include, but not limit to the monomer drugs extracted from plants and animals used in the chemically cure drugs. These chemically synthesized drugs, antibiotics or biochemical drugs may be used solely or used as a complex of several above-mentioned chemically synthesized drugs, antibiotics or biochemical drugs.

Said matrix adjuvants of this invention include the adjuvants mainly derived from natural materials, especially from plants. The matrix adjuvants may optionally include a plastifying component.

More specifically, said matrix adjuvant of this invention is at least one of the adjuvants selected from a group consisting of the following: a pharmaceutically acceptable monosaccharide, oligosaccharide, polysaccharide, sugar ester, sugar alcohol, alpha-hydroxy acid (fruit acid), higher fatty acid derivative, higher aliphatic alcohol, polyol, urea, poly(ethylene oxide) derivative.

For the above-mentioned substances, non-limiting examples of said monosaccharide include D-ribose, fructose, glucose, xylose; examples of said oligosaccharide include trehalose, raffinose, maltose; example of said polysaccharide includes gelose; examples of said sugar ester include sucrose ester, D-ribonic acid-γ-lactone; examples of said sugar alcohol include erythritol, sorbitol, xylitol, arabitol, isomaltitol, lactitol; examples of said alpha-hydroxy acid include malic acid, citric acid; examples of said higher fatty acid derivative include sodium stearate, glycerin stearate, glycerin palmitate, shellac; examples of said higher aliphatic alcohol include cetyl alcohol, stearyl alcohol; example of said polyol includes phenyl ethanediol; examples of said poly(ethylene oxide) derivative include polyoxyethylene monosteatate, polyoxyethylene alkyl ether, and the above-mentioned compounds containing crystal water.

For the above-mentioned substances, said materials derived from plants include erythritol, sorbitol, fructose, D-ribonic acid-γ-lactone, arabitol, trehalose, D-ribose, low-melting point gelose, shellac, xylitol, raffinose, glucose, malic acid, citric acid, isomaltitol, lactitol, maltose, xylose, sucrose ester, etc., and the above-mentioned compounds containing crystal water; examples of said chemically synthesized adjuvants or adjuvants derived from animals include phenyl ethanediol, polyethylene glycol, cetyl alcohol, stearyl alcohol, sodium stearate, glycerin stearate, glycerin palmitate, urea, polyoxyethylene monosteatate, polyoxyethylene alkyl ether.

For the above-mentioned substances, one or more adjuvants selected from sorbitol, lactitol, maltose, sucrose ester, and the above-mentioned compounds containing crystal water are most preferred.

The matrix adjuvants are natural adjuvants mainly derived from plants, it means that the content of the adjuvants derived from plants is equal to or more than 50wt% and the content of the chemically synthesized adjuvants and the adjuvants derived from animals are less than or equal to the adjuvants derived from plants in the matrix adjuvants. Preferably, only the adjuvants derived from plants is used for the matrix adjuvants of the drop pills, or the adjuvant derived from plants is the main component and only small amount of the chemically synthesized adjuvants and the adjuvants derived from animals are used. The content of the chemically synthesized adjuvants and the adjuvants derived from animals was less than 50wt%, preferably less than 40wt%, and more preferably less than 30wt%. Said natural adjuvant derived from plants refers to the following: the adjuvant itself is extracted from cells or tissues of the plants, or the products obtained by modification of the extract of plants, such as derivation and the like. Said chemically synthesized adjuvants refer to the artificial synthesized small molecule compounds or polymers obtained by chemical synthesis from the simple small molecules. Said natural adjuvant derived from animals refers to the following: the adjuvant itself is extracted from cells or tissues of the animals, or the products obtained by modification of the extract of animals, such as derivation and the like.

Said matrix adjuvants derived from plants may have or will have artificial synthetic products in the future. If the artificial synthetic products have identical or similar properties to the natural matrix adjuvants derived from plants, for example, they have the characteristics such as safety and non-toxicity, they may be applied as the substitutes of the natural matrix adjuvants derived from plants, just like the application of the natural matrix adjuvants derived from plants.

In order to improve the shaping ability of the drop pill, preferably, the matrix further comprises plastifying components. Said plastifying components may be one or more components selected from a group consisting of the following: natural adjuvants derived from plants such as starch and the derivatives thereof, cellulose and the derivatives thereof, arabic gum, dextran, chitin, sesbania gum, carrageen gum, Indian gum, danish agar, tragacanth gum, carrageenin, tamarind gum, pectin, xanthan gum, alginic acid and the salts thereof, dextrin, cyclodextrin, agar, lactose; the adjuvants chemically synthesized and derived from animals such as polyvinylpyrrolidone, cross-linked polyvinylpyrrolodione, carbomer, polyvinyl alcohol, acrylic acid resin, poloxamer, silicon dioxide, glutin, and the like.

Non-limiting examples of said starch and their derivatives include pregelatinized starch, modified starch, hydroxypropyl starch, carboxylmethyl starch and the like. Non-limiting examples of said cellulose and their derivatives include methyl cellulose, microcrystalline cellulose, sodium carboxylmethyl cellulose, hydroxypropyl methyl cellulose, cross-linked sodium carboxylmethyl cellulose, hydroxyethyl methyl cellulose, hydroxyethyl cellulose, hydroxypropyl cellulose.

Preferably, said plastifying components may be one or more components selected from a group consisting of the following: pregelatinized starch, carboxylmethyl starch, methyl cellulose, sodium carboxylmethyl cellulose, hydroxypropyl methyl cellulose, arabic gum, alginic acid, dextrin, cyclodextrin, agar, and lactose. In addition, glycerin monostearate and polyoxyethylene monostearate may also be used as plastifying components together with other matrix adjuvants.

Said components derived from plants refer to the following: the adjuvant itself is extracted from cells or tissues of the plants, or obtained by modification, such as derivation and the like, of the extract of plants. Said chemically synthesized adjuvants refer to the artificial synthesized small molecule compounds or polymers obtained by chemical synthesis of simple small molecules. Said natural adjuvant derived from animals refers to the following: the adjuvant itself is extracted from cells or tissues of the animals, or obtained by modification, such as derivation and the like, of the extract of animals.

Perhaps, said plastifying components derived from plants have had or will have artificial synthetic products in the future. If the artificial synthetic products have identical or similar properties to the natural plastifying components derived from plants and have the characteristics such as safe and non-toxic, they may be applied as the substitutes for the natural plastifying components derived from plants, just like the application of the natural plastifying components derived from plants.

Said matrix adjuvants selected from monosaccharide, oligosaccharide, polysaccharide, sugar ester, sugar alcohol, alpha-hydroxy acid, higher fatty acid derivative, higher aliphatic alcohol, polyol, urea, poly(ethylene oxide) derivative, etc., preferably, the matrix adjuvants derived from plants are selectively combined with said plastifying components according to the characteristics of drugs. The preferred combination include, but are not limited to, the following: xylitol and starch; lactitol and starch; xylitol and arabic gum; sugar ester and glycerin monostearate; sugar ester and polyoxyethylene monostearate; sugar ester, polyoxyethylene monostearate and cross-linked sodium carboxylmethyl cellulose; sucrose ester, polyoxyethylene monostearate, cross-linked sodium carboxyl methyl cellulose and silicon dioxide.

The weight ratio of said matrix adjuvant and plastifying component is 1:0∼1:1.5; preferably 1:0.1∼1:0.9; most preferably 1:0.1∼1:0.5.

For said matrix adjuvant, the preferred weight ratio of xylitol and starch is 1:0.2∼1:0.3.

For said matrix adjuvant, the preferred weight ratio of lactitol and starch is 1:0.2∼1:0.3.

For said matrix adjuvant, the preferred weight ratio of xylitol and arabic gum is 1:0.2∼1:0.4.

For said matrix adjuvant, the weight ratio of sugar ester and glycerin monostearate is 1:0.1∼1:1, most preferably 1:0.5.

For said matrix adjuvant, the weight ratio of sugar ester and polyoxyethylene monostearate is 1:0.1∼1:1, most preferably 1:0.5.

For said matrix adjuvant, the weight ratio of sugar ester, polyoxyethylene monostearate and cross-linked sodium carboxylmethyl cellulose is 1:(0.1∼1):(0.1∼1), most preferably 1:0.4:0.6.

For said matrix adjuvant, the weight ratio of sucrose ester, polyoxyethylene monostearate, cross-linked sodium carboxylmethyl cellulose and silicon dioxide is 15:(7∼15):(0.1∼2):(0.1∼2), most preferably 15:11:1:1.

The weight ratio of matrix adjuvant of the drop pills and active ingredient is 1:0.1∼1:1, more preferably 1:0.1∼1:0.6, most preferably 1:0.2∼1:0.4.

One or more said matrix adjuvants are applied in the drop pills of this invention, the weight ratio of the matrix adjuvant and the active ingredient meets the requirements above-mentioned, and the weight ratio of the matrix adjuvant and the plastifying component also meets the above-mentioned requirements.

The preparing process of the drop pills may use the conventional method, for example, the drop pills may be prepared according to following procedures:
a. To select one or more matrix adjuvants from the above mentioned, or select at least one matrix adjuvants as well as one or more said plastifying components, and to mix them homogeneously;
b. To transfer the above homogenized matrix adjuvant(s) or the matrix adjuvant mixture into a dropping machine, add active ingredients, and stir said mixture of matrix adjuvant(s) and active ingredients for homogenization;
c. To heat the mixture obtained from step b to melt, drop the melted mixture into the coolant, and filter the drop pills after solidification;
d. To wipe off or centrifuging the coolant on the surface of drop pills;
e. To dry the wiped drop pills at a low temperature, and obtain the said drop pills.

In said preparing process of the drop pills, the weight ratio of matrix adjuvant and the plastifying component is 1:0∼1.5, preferably 1:0.1∼0.9, most preferably 1:0.1∼0.5. The aim of adding plastifying component is to improve the inner cohesion and plasticity of the drop pills. Whether adding said plastifying component or not mainly depends on the property of the active ingredient. If the active ingredient itself possesses good plasticity, and has the property of inner cohesion, no plastifying component is needed, or much less plastifying component is needed. If not, a certain amount of plastifying component is needed.

In said preparing process of the drop pills, the weight ratio of the matrix adjuvant and the active ingredients is 1:0.1∼1:1, more preferably 1:0.6∼1:06, most preferably 1:0.2∼1:0.4.

In said preparing process of the drop pills, the stirring time for mixing the active ingredients with the matrix adjuvants is 10-30 minutes; After homogenized, the heating temperature for melting or dropping the obtained mixture is 45∼95°C, more preferably 60∼95°C. Examples of coolant are liquid paraffin, methyl silicone oil or vegetable oil including bean oil, castor oil, and the like, etc., and liquid paraffin, methyl silicone oil are preferred. The temperature of the coolant is -20∼30°C, preferably 0∼18°C. The inner diameter of the dropper is 1.0∼4.0mm, preferably 1.2∼2.5mm. Less difference between the outer diameter and the inner diameter of the dropper is preferred.

It should be noted that the invention will be better understood by reference to the above content which illustrate but do not limit the preparing process of the drop pills in any way.

In addition to the advantages of the conventional drop pills, such as easy preparation, stable quality, solidifying the liquid active ingredient, convenient administration, and high and quick efficacy, the greatest advantage of the drop pills prepared in the present invention lies in the following: the matrix adjuvants used in the present invention are derived from the natural plants, or mainly comprises the matrix adjuvant derived from the natural plants. The matrix adjuvants derived from the natural plants are not only pharmaceutically acceptable, but are commonly used as additives in the food industry. Since said matrix adjuvant is not only absolutely safe without any toxic and side effects, but also very cheap and accessible, it has great value for application and popularization, thus laying a solid foundation for the internationalization of said drop pills.

Hereinafter the present invention is further explained by way of examples, but the present invention should not be limited by examples in any way.

Unless otherwise specified, the medicinal materials, extracts from medicinal materials and chemical drugs referred in following examples are commercially available products or prepared by the conventional method in the art, and all reagents used are commercially available.

### Examples

### Example 1:

*Radix Puerariae daidzein* 20g, trehalose 35g, dextrine 20g.

Alga alcohol and dextrine were fully mixed and transferred to a dropping machine, to which *Radix Puerariae daidzein* was added. The mixture was stirred to homogenize, then heated to be molten using a water-bath at temperature of 90°C. Dropping the melted mixture at temperature of 80°C into coolant of liquid paraffin at temperature of 4°C at the speed of 35 pellets/min, After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 5.14min without baffle, which complied with the requirements of the Chinese Pharmacopoeia.

### Example 2:

Extract derived from *Radix Ginseng*, *Radix Ophiopogonis*, *Fructus Schisandrae* in the weight ratio of 1:2:1.

Said extract 15g. arabitol 35g, hydroxypropyl methyl cellulose 12g, xanthan gum 6g.

Arabitol, hydroxypropyl methyl cellulose and xanthan gum, were fully mixed and transferred to a dropping machine, to which said extract was added. The mixture was stirred to homogenize, then heated to be molten using a water-bath at temperature of 90°C. Dropping the melted mixture at temperature of 80°C into coolant of liquid paraffin at temperature of 8°C at the speed of 40 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 2.98min without baffle, which complied with the requirements of the Chinese Pharmacopoeia.

### Example 3:

13g extracts of *Radix Salviae Miltiorrhizae*, 5g extracts of *Radix Notoginseng* (WO 02/058625 A2), borneol 1.2g, lactitol 45g, pregelatined starch 12g.

Lactitol and starch were fully mixed and transferred to a dropping machine, to which the extracts of *Radix Salviae Miltiorrhizae,* the extracts of *Radix Notoginseng* and borneol were added. The mixture was stirred to homogenize, then heated to be molten using a water-bath at temperature of 83°C. Dropping the melted mixture at temperature of 70°C into coolant of methyl silicone oil at temperature of 0°C at the speed of 35 pellets/min. After shaping, the methyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.96min without baffle, which complied with the requirements of the Chinese Pharmacopoeia.

### Example 4:

*Radix Paeoniae Alba*, *Herba Ephedrae* and *Radix Glycyrrhizae* were extracted with water; *Rhizoma Pinelliae Preparata*, *Rhizoma Zingiberis*, and *Fructus Schisandrae Chinensis* were extracted with ethanol. The above two extracting solutions were combined and concentrated to be extracts; the volatile oil was obtained by distilling of *Herba Asari*, *Ramulus Cinnamomi*, respectively.

Said extracts 16g, arabitol 20g, carboxyl methyl starch 16g.

Lactitol and carboxyl methyl starch were fully mixed and transferred to a dropping machine, to which the extracts and volatile oil were added. The mixture was stirred to homogenize, then heated to be molten using a water-bath at temperature of 80°C. Dropping the melted mixture at temperature of 70°C into coolant of liquid paraffin at temperature of 4°C at the speed of 40 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.10min without baffle, which complied with the requirements of the Chinese Pharmacopoeia.

### Example 5:

*Rhizoma Coptidis*, *Cortex Phellodendri*, *Fructus Gardeniae*, and *Radix Scutellariae* with the weight ratio of 1.6:1.1:1.1:2.2 were extracted with water, precipitating with alcohol, then concentrated to obtain the extract.

Said extract 12g. xylitol 30g, methyl cellulose 18g, starch 5g.

Xylitol and methyl cellulose were fully mixed and transferred to a dropping machine, to which the extract was added. The mixture was stirred to homogenize, then heated to be molten using a water-bath at temperature of 90°C. Dropping the melted mixture at temperature of 85°C into coolant of liquid paraffin at temperature of 10°C at the speed of 30 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.76min without baffle, which complied with the requirements of the Chinese Pharmacopoeia.

### Example 6:

Extract of leech (*Whitmania pigra Whitman*) 20g, sorbitol 40g, methyl cellulose 15g.

Sorbitol and methyl cellulose were fully mixed and transferred to a dropping machine, to which the extract of leech was added. The mixture was stirred to homogenize, then heated to be molten using a water-bath at temperature of 85°C. Dropping the melted mixture at temperature of 80°C into coolant of liquid paraffin at temperature of 8°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 4.10min without baffle, which complied with the requirements of the Chinese Pharmacopoeia.

### Example 7:

*Radix Bupleuri* was extracted with hot water, precipitating with alcohol, then concentrated to obtain the extract.

Said extract 18g, xylitol 35g, starch 12g.

Xylitol and starch were fully mixed and transferred to a dropping machine, to which the extract was added. The mixture was stirred to homogenize, then heated to be molten using a water-bath at temperature of 85°C. Dropping the melted mixture at temperature of 75°C into coolant of methyl silicone oil at temperature of 4°C at the speed of 35 pellets/min. After shaping, the methyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3,25min without baffle, which complied with the requirements of the Chinese Pharmacopoeia.

### Example 8:

The volatile oil of *Radix Bupleuri* was obtained by method of supercritical extraction, the residue was extracted to obtain saikosaponin as the effective component, and then the extract was obtained.

Said extract 20g, maltose 40g, carboxyl methyl cellulose 10g.

Lactitol and carboxyl methyl cellulose were fully mixed and transferred to a dropping machine, to which the extract and the volatile oil were added. The mixture was stirred to homogenize, then heated to be molten using a water-bath at temperature of 85°C. Dropping the melted mixture at temperature of 70°C into coolant of liquid paraffin at temperature of 5°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 4.15min without baffle, which complied with the requirements of the Chinese Pharmacopoeia.

### Example 9:

Oil of *Blumea balsamifera (L.) DC.* 14g, borneol 1g, lactitol 35g, arabic gum 20g.

Lactitol and arabic gum were fully mixed and transferred to a dropping machine, to which oil of *Blumea balsamifera (L.) DC.* and borneol were added. The mixture was stirred to homogenize, then heated to be molten using a water-bath at temperature of 70°C. Dropping the melted mixture at temperature of 60°C into coolant of liquid paraffin at temperature of 0°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.43min without baffle, which complied with the requirements of the Chinese Pharmacopoeia.

### Example 10:

Extract of *Radix Salviae Miltiorrhizae* and *Radix Notoginseng* (Chinese Patent No. CN 1348815A) 12g, borneol 1.2g, xylitol 40g, starch 8g.

Xylitol and starch were fully mixed and transferred to a dropping machine, to which extracts of *Radix Salviae Miltiorrhizae* and *Radix Notoginseng* and borneol were added. The mixture was stirred to homogenize, then heated to be molten using a water-bath at temperature of 80°C. Dropping the melted mixture at temperature of 64°C into coolant of methyl silicone oil at temperature of 04°C at the speed of 40 pellets/min. After shaping, the methyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.63min without baffle, which complied with the requirements of the Chinese Pharmacopoeia,

### Example 11:

Extract of *Radix Puerariae* (the content of pueraria flavones is more than 80%) 15g, lactitol 25g, carrageen gum 20g, starch 6g.

Lactitol, carrageen gum and starch were fully mixed and transferred to a dropping machine, to which extract of *Radix Puerariae* was added. The mixture was stirred to homogenize, then heated to be molten using a water-bath at temperature of 90°C. Dropping the melted mixture at temperature of 85°C into coolant of methyl silicone oil at temperature of 10°C at the speed of 40 pellets/min. After shaping, the methyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.64min without baffle, which complied with the requirements of the Chinese Pharmacopoeia.

### Example 12:

Extract of *Radix Puerariae* (the content of pueraria flavones is more than 40%, and the content of puerarin is more than 28%)15g, fructose 25g, carrageen gum 20g, starch 6g.

Lactitol, carrageen gum and starch were fully mixed and transferred to a dropping machine, to which extract of *Radix Puerariae* was added. The mixture was stirred to homogenize, then heated to be molten using a water-bath at temperature of 90°C. Dropping the melted mixture at temperature of 85°C into coolant of liquid paraffin at temperature of 10°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.15min without baffle, which complied with the requirements of the Chinese Pharmacopoeia.

### Example 13:

*Oleum Rhododendri Daurici* 13g, isomaltitol 20g, alginic acid 15g.

Isomaltitol and alginic acid were fully mixed and transferred to a dropping machine, to which *Oleum Rhododendri Daurici* was added. The mixture was stirred to homogenize, then heated to be molten using a water-bath at temperature of 80°C. Dropping the melted mixture at temperature of 70°C into coolant of liquid paraffin at temperature of 4°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.78min without baffle, which complied with the requirements of the Chinese Pharmacopoeia.

### Example 14:

*Oleum Viticis Negundo* 12g, isomaltitol 35g, carrageen gum 12g. Isomaltitol and carrageen gum were fully mixed and transferred to a dropping machine, to which *Oleum Viticis Negundo* was added. The mixture was stirred to homogenize, then heated to be molten using a water-bath at temperature of 80°C. Dropping the melted mixture at temperature of 65°C into coolant of liquid paraffin at temperature of 0°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.55min without baffle, which complied with the requirements of the Chinese Pharmacopoeia.

### Example 15:

Extract of *Radix Salviae Miltiorrhizae* and *Radix Notoginseng* (Chinese Patent No. CN 1348815A) 22g, borneol 1.5g, lactitol 40g, arabic gum 15g.

Lactitol and arabic gum in the formulation were fully mixed and transferred to a dropping machine, to which extracts of *Radix Salviae Miltiorrhizae* and *Radix Notoginseng* and borneol were added. The mixture was stirred to homogenize, then heated to be molten using a water-bath at temperature of 85°C. Dropping the melted mixture at temperature of 64°C into coolant of liquid paraffin at temperature of 4°C at the speed of 40 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 4.25min without baffle, which complied with the requirements of the Chinese Pharmacopoeia.

### Example 16:

Extracts derived from *Flos Lonicerae*, *Radix Scutellariae*, *Fructus Forsythia* with the weight ratio of 1:1:2,

Said extracts 20g, arabitol 35g, cyclodextrin 15g.

Aarabitol and dextrin were fully mixed and transferred to a dropping machine, to which the extract was added. The mixture was stirred to homogenize, then heated to be molten using a water-bath at temperature of 95°C. Dropping the melted mixture at temperature of 80°C into coolant of liquid paraffin at temperature of 10°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.68min without baffle, which complied with the requirements of the Chinese Pharmacopoeia.

### Example 17:

Styrallyl ester 12g, borneol 0.5g, xylitol 40g, hydroxypropyl methyl cellulose 10g.

Xylitol and hydroxypropyl methyl cellulose were fully mixed and transferred to a dropping machine, to which styrallyl ester and borneol were added. The mixture was stirred to homogenize, then heated to be molten using a water-bath at temperature of 75°C. Dropping the melted mixture at temperature of 60°C into coolant of liquid paraffin at temperature of 0°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.10min without baffle, which complied with the requirements of the Chinese Pharmacopoeia.

### Example 18:

The volatile oil of *Rhizoma Chuanxiong* was obtained by method of supercritical extraction, the residue was extracted with the low concentration ethanol, and concentrated to obtain the extract.

Said extract 12g, borneol 0.5g, lactitol 35g, alginic acid 15g.

Lactitol and alginic acid were fully mixed and transferred to a dropping machine, to which the extract, volatile oil and borneol were added. The mixture was stirred to homogenize, then heated to be molten using a water-bath at temperature of 85°C. Dropping the melted mixture at temperature of 65°C into coolant of liquid paraffin at temperature of 0°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.22min without baffle, which complied with the requirements of the Chinese Pharmacopoeia.

### Example 19:

The extract of *Erigeron brevsicapus (Vant) Hand-Mazz*, *Folium Ginkgo*, *Radix Salviae Miltorrhizae* and natural borneol was prepared according to the method of yinzhanxinmai drop pills in «National Specification Compilation of Traditional Chinese Patent Medicines Preparations», Part of Internal medicine and Heart.

Said extract 15g, arabitol 40g, dextrin 12g, xanthan gum 5g.

Arabitol, dextrin and xanthan gum were fully mixed and transferred to a dropping machine, to which the extract and borneol were added. The mixture was stirred to homogenize, then heated to be molten using a water-bath at temperature of 90°C, Dropping the melted mixture at temperature of 75°C into coolant of methyl silicone oil at temperature of 8°C at the speed of 35 pellets/min. After shaping, the methyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 4.16min without baffle, which complied with the requirements of the Chinese Pharmacopoeia.

### Example 20:

The ethyl ether extract of *Rhizoma Chuanxiong.* 7.5g; the ethyl ether extract of *Radix Angelicae Sinensis*: 13.5g; D-ribonic acid-γ-lactone 30g; carrageen gum 12g.

Xylitol and carrageen gum were fully mixed and transferred to a dropping machine, to which the ethyl ether extract of *Rhizoma Chuanxiong* and *Radix Angelicae Sinensis* were added. The mixture was stirred to homogenize, then heated to be molten using a water-bath at temperature of 85°C. Dropping the melted mixture at temperature of 75°C into coolant of liquid paraffin at temperature of 4°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.35min without baffle, which complied with the requirements of the Chinese.

### Example 21:

*Sonqi tofal Saponins std.* 12g, xylitol 35g, lactose 12g, arabic gum 5g.

Xylitol, lactose and arabic gum were fully mixed and transferred to a dropping machine, to which *Sonqi tofal Saponins std.* was added. The mixture was stirred to homogenize, then heated to be molten using a water-bath at temperature of 85°C. Dropping the melted mixture at temperature of 75°C into coolant of liquid paraffin at temperature of 10°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 4.65min without baffle, which complied with the requirements of the Chinese Pharmacopoeia.

### Example 22:

Essential oil of *Cinnamomum migao H. W. Li* extracted by the water vapor distillation: 5g; extract of *Bulbus Alli Macrostemi* with ethanol: 4g; natural borneol 2g; sorbitol 30g; alginic acid 15g.

Sorbitol and alginic acid were fully mixed and transferred to a dropping machine, to which essential oil of *Cinnamomum migao H. W. Li,* extract of *Bulbus Alli Macrostemi and* natural borneol were added. The mixture was stirred to homogenize, then heated to be molten using a water-bath at temperature of 80°C. Dropping the melted mixture at temperature of 75°C into coolant of liquid paraffin at temperature of 0°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.78min without baffle, which complied with the requirements of the Chinese Pharmacopoeia.

### Example 23:

Andrographolide 15g, xylitol 40g, hydroxypropyl methyl cellulose 13g, starch 8g.

Xylitol, hydroxypropyl methyl cellulose and starch were fully mixed and transferred to a dropping machine, to which andrographolide was added. The mixture was stirred to homogenize, then heated to be molten using a water-bath at temperature of 90°C. Dropping the melted mixture at temperature of 80°C into coolant of liquid paraffin at temperature of 4°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 4.96min without baffle, which complied with the requirements of the Chinese Pharmacopoeia.

### Example 24:

Cyclovirobuxine D 12g, lactitol 30g, tragacanth 15g.

Lactitol and tragacanth were fully mixed and transferred to a dropping machine, to which cyclovirobuxine D was added. The mixture was stirred to homogenize, then heated to be molten using a water-bath at temperature of 90°C. Dropping the melted mixture at temperature of 85°C into coolant of liquid paraffin at temperature of 10°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 4.23min without baffle, which complied with the requirements of the Chinese Pharmacopoeia.

### Example 25:

Tinidazole 8g, sorbitol 30g, polyvinylpyrrolidone 10g.

Sorbitol and polyvinylpyrrolidone were fully mixed and transferred to a dropping machine, to which tinidazole was added. The mixture was stirred to homogenize, then heated to be molten using a water-bath at temperature of 90°C. Dropping the melted mixture at temperature of 80°C into coolant of liquid paraffin at temperature of 8°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 5.62min without baffle, which complied with the requirements of the Chinese Pharmacopoeia.

### Example 26:

Flavones of *Scabisa Comosa Fisch* extracted with 60% ethanol: 15g; lactitol 40g; chitin 12g; xanthan gum 5g.

Lactitol, chitin and xanthan gum were fully mixed and transferred to a dropping machine, to which flavones of *Scabisa Comosa Fisch* was added. The mixture was stirred to homogenize, and then heated to be molten using a water-bath at temperature of 90°C. Dropping the melted mixture at temperature of 75°C into coolant of liquid paraffin at temperature of 4°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 4.72min without baffle, which complied with the requirements of the Chinese Pharmacopoeia.

### Example 27:

Rutin 10g, shellac 35g, hydroxypropyl methyl cellulose 15g.

Lactitol and hydroxypropyl methyl cellulose were fully mixed and transferred to a dropping machine, to which rutin was added. The mixture was stirred to homogenize, and then heated to be molten using a water-bath at temperature of 90°C. Dropping the melted mixture at temperature of 85°C into coolant of methyl silicone oil at temperature of 8°C at the speed of 40 pellets/min. After shaping, the methyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 5.43min without baffle, which complied with the requirements of the Chinese Pharmacopoeia.

### Example 28:

Extract of *Polygala tenuifolia Willd.* was prepared according to the method of polygala tincture described in Chinese Pharmacopoeia 2000 (1^{st} Supplement).

Said extract 12g, raffinose 30g, tragacanth 12g.

Xylitol, and tragacanth were fully mixed and transferred to a dropping machine, to which the extract was added. The mixture was stirred to homogenize, and then heated to be molten using a water-bath at temperature of 90°C. Dropping the melted mixture at temperature of 75°C into coolant of liquid paraffin at temperature of 4°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 4.38min without baffle, which complied with the requirements of the Chinese Pharmacopoeia.

### Example 29:

Extract of *Cortex Moutan* and *Rhizome Chuanxiong* was prepared according to the method of suxiaoxintong drop pills described in National Specification Compilation of Traditional Chinese Patent Medicines Preparations, 19th Volume.

Said extract 15g, sorbitol 35g, starch 20g.

Sorbitol and starch were fully mixed and transferred to a dropping machine, to which the extract and borneol were added. The mixture was stirred to homogenize, and then heated to be molten using a water-bath at temperature of 90°C. Dropping the melted mixture at temperature of 80°C into coolant of liquid paraffin at temperature of 5°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.42min without baffle, which complied with the requirements of the Chinese Pharmacopoeia.

### Example 30:

Distillate was obtained by refluxing or distilling *Radix Bupleuri,* then the distillate was loaded to the column of macroporous absorption resin, and then eluting the column with ethanol, and the eluant was concentrated to obtain the volatile oil of *Radix Bupleuri.*

Volatile oil of *Radix Bupleuri*, 15g; lactitol. 35g; carrageen gum, 20g.

Lactitol and carrageen gum were fully mixed and transferred to a dropping machine, to which the volatile oil of *Radix Bupleuri* was added. The mixture was stirred to homogenize, and then heated to be molten using a water-bath at temperature of 80°C. Dropping the melted mixture at temperature of 65°C into coolant of liquid paraffin at temperature of 4°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 2.98min without baffle, which complied with the requirements of the Chinese Pharmacopoeia.

### Example 31:

Extract of *Radix Platycodi*, *Polygala tenuifolia Willd*,, *Flos Farfarae*, *Radix Glycyrrhizae* was prepared according to the method of *Table of Platycodon powder compound* in Pharmaceutical Specification Compilation of Traditional Chinese Patent Medicines Preparations of Ministry of Public Health, 4th Volume.

Said extract 18g, maltose 40g, carboxyl methyl starch 12g, polyoxyethylene alkyl ether 6g,

Xylitol and carboxyl methyl starch were fully mixed and transferred to a dropping machine, to which the extract was added. The mixture was stirred to homogenize, and then heated to be molten using a water-bath at temperature of 90°C. Dropping the melted mixture at temperature of 82°C into coolant of methyl silicone oil at temperature of 6°C at the speed of 35 pellets/min. After shaping, the methyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 5.32min without baffle, which complied with the requirements of the Chinese Pharmacopoeia.

### Example 32:

Thymol 14g, clove oil 2.25g, sorbitol 45g. methyl cellulose 15g.

Sorbitol and methyl cellulose were fully mixed and transferred to a dropping machine, to which thymol and clove oil were added. The mixture was stirred to homogenize, and then heated to be molten using a water-bath at temperature of 70°C. Dropping the melted mixture at temperature of 60°C into coolant of liquid paraffin at temperature of 0°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.74min without baffle, which complied with the requirements of the Chinese Pharmacopoeia.

### Example 33:

The weight ratio of *Radix Aconiti Lateralis Preparata, Radix Glycyrrhizae*, and *Rhizoma Zingiberis* was 1:1:9, *Radix Aconiti Lateralis Preparata* was extracted with acidic aqueous solution, precipitated with alcohol, and then the extract was concentrated and dried in vacuum; *Radix Glycyrrhizae* was extracted with dilute ammonia aqueous solution, the extract was concentrated, precipitated with acid, centrifuged to obtain the precipitate, the precipitate was crushed and sifted; the extract of the mixture of the above two. The volatile oil of *Rhizoma Zingiberis* was obtained by water vapor distillation.

Said extracted mixture 14g, D-ribose 35g, agar 15g, arabic gum 5g.

D-ribose, starch and arabic gum were fully mixed and transferred to a dropping machine, to which the extracted mixture and the volatile oil of *Rhizoma Zingiberis* were added. The mixture was stirred to homogenize, and then heated to be molten using a water-bath at temperature of 85°C. Dropping the melted mixture at temperature of 75°C into coolant of liquid paraffin at temperature of 3°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutinafion, Determination results of disintegration time according to the method described in Chinese Pharmacopoeia (2000) revealed that the drop pills passed the wire mesh completely within the mean time of 5.22min without baffle, which complied with the requirements of the Chinese Pharmacopoeia.

### Example 34:

Extract of *Cortex Cinnamomi*, *Rhizoma Chuanxiong*, *Rhizoma Cyperi* was prepared according to the method of xintongning drop pills in Pharmaceutical Specification Compilation of Traditional Chinese Patent Medicines Preparations of Ministry of Public Health, 15th Volume.

Said extract 12g, erythritol 14g, starch 15. polyvinylpyrrolidone 5g.

Erythritol and starch were fully mixed and transferred to a dropping machine, to which the extract was added. The mixture was stirred to homogenize, and then heated to be molten using a water-bath at temperature of 85°C. Dropping the melted mixture at temperature of 75°C into coolant of liquid paraffin at temperature of 4°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 4.15min without baffle, which complied with the requirements of the Chinese Pharmacopoeia.

### Example 35:

Extract of *Boswellia carteri*, *Lignum Santali Album*, *Radix Aristolochiae* was prepared according to the method of guanxinsuhe drop pills in Pharmaceutical Specification Compilation of Traditional Chinese Patent Medicines Preparations of Ministry of Public Health, 12th Volume.

Said extract 10g, sorbitol 32g, carrageen gum 18g, starch 5g.

Sorbitol, carrageen gum and starch were fully mixed and transferred to a dropping machine, to which the extract, styrax and borneol were added. The mixture was stirred to homogenize, and then heated to be molten using a water-bath at temperature of 80°C. Dropping the melted mixture at temperature of 65°C into coolant of liquid paraffin at temperature of 0°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 2.68min without baffle, which complied with the requirements of the Chinese Pharmacopoeia.

### Example 36:

Volatile oil of *Artemisia subdigitata Mattf.* obtained by the method of water vapor distillation: 18g; xylitol 40g; alginic acid 13g.

Xylitol and alginic acid were fully mixed and transferred to a dropping machine, to which the volatile oil of *Artemisia subdigitata Mattf.* was added. The mixture was stirred to homogenize, and then heated to be molten using a water-bath at temperature of 80°C. Dropping the melted mixture at temperature of 65°C into coolant of liquid paraffin at temperature of 4°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.16min without baffle, which complied with the requirements of the Chinese Pharmacopoeia.

### Example 37:

12g of the volatile oil of *Rhododendron lutescens Franch.* was obtained by the method of water vapor distillation, citric acid 35g, Indian gum 10g, polyoxyethylene monostearate 10g.

Xylitol and carrageen gum were fully mixed and transferred to a dropping machine, to which the volatile oil of *Rhododendron lutescens Franch.* was added. The mixture was stirred to homogenize, and then heated to be molten using a water-bath at temperature of 80°C. Dropping the melted mixture at temperature of 65°C into coolant of liquid paraffin at temperature of 0°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.75min without baffle, which complied with the requirements of the Chinese Pharmacopoeia.

### Example 38:

Extract of *Radix Sophorae Tonkinensis*, *Cortex Phellodendri*, *Radix Trichosanthis*, *Radix Arisfolochiae*, *Radix Angelicae Dahuricae* and *Herba Asari* was prepared according to the preparation method of yatongning drop pill described in National Specification Compilation of Traditional Chinese Patent Medicines Preparations, Part of Internal medicine and Stomatology.

Said extract 12g, lactitol 35g, dextrin10g.

Lactitol and dextrin were fully mixed and transferred to a dropping machine, to which the extract, camphor and natural borneol were added. The mixture was stirred to homogenize, and then heated to be molten using a water-bath at temperature of 90°C. Dropping the melted mixture at temperature of 80°C into coolant of methyl silicone oil at temperature of 8°C at the speed of 35 pellets/min. After shaping, the methyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time according to the method described in Chinese Pharmacopoeia (2000) revealed that the drop pills passed the wire mesh completely within the mean time of 3.10min without baffle, which complied with the requirements of the Chinese Pharmacopoeia.

### Example 39:

Extract of *Folium Ginkgo*, *Radix Salviae Miltiorrhizae*, *Gynostemma pentaphyllum* was prepared according to the preparation method of Yindanxintai drop pill described in National Specification Compilation of Traditional Chinese Patent Medicines Preparations, Part of Internal medicine and Heart.

Said extract 13g, alginic alcohol 40g, hydroxypropyl methyl cellulose 15g, xanthan gum 5g.

Alginic alcohol, hydroxypropyl methyl cellulose and xanthan gum were fully mixed and transferred to a dropping machine, to which the extract and natural borneol were added. The mixture was stirred to homogenize the mixture, and then heated to be molten using a water-bath at temperature of 90°C. Dropping the melted mixture at temperature of 75°C into coolant of liquid paraffin at temperature of 10°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 4.93min without baffle, which complied with the requirements of the Chinese Pharmacopoeia.

### Example 40:

Chlorphenamine maleate 8g, isomaltose 45g, alginic acid 25g.

Isomaltose and alginic acid were fully mixed and transferred to a dropping machine, to which Chlorphenamine maleate was added. The mixture was stirred to homogenize the mixture, and then heated to be molten using a water-bath at temperature of 90°C. Dropping the melted mixture at temperature of 82°C into coolant of liquid paraffin at temperature of 10°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 5.14min without baffle, which complied with the requirements of the Chinese Pharmacopoeia.

### Example 41:

Bifendate 4g, isomaltitol, 30g, xanthan gum 3g, poloxamer 6g.

Isomaltitol and xanthan gum were fully mixed and transferred to a dropping machine, bifendate was dissolved with an appropriate amount of 95% ethanol and added to the above mixture. The mixture was stirred to homogenize, heated to be molten using a water-bath at temperature of 95°C. Dropping the melted mixture at temperature of 90°C into coolant of liquid paraffin at temperature of 5°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 5.33min without baffle, which complied with the requirements of the Chinese Pharmacopoeia.

### Example 42:

*Salvia miltiorrhiza Bunge* was extracted with hot water, the extracted solution was loaded to resin column, eluted with alcohol, and the eluate was concentrated to obtain extract of *Salvia miltiorrhiza Bunge.*

Extract of *Salvia miltiorrhiza Bunge,* 15g; xylitol, 35g; tragacanth. 15g.

Xylitol and tragacanth were fully mixed and transferred to a dropping machine, to which the extract of *Salvia miltiorrhiza Bunge* was added. The mixture was stirred to homogenize, heated to be molten using a water-bath at temperature of 90°C. Dropping the melted mixture at temperature of 75°C into coolant of liquid paraffin at temperature of 8°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.65min without baffle, which complied with the requirements of the Chinese Pharmacopoeia.

### Example 43:

Phenobarbital 6g, xylitol 30g, lactose 15g, xanthan gum 5g.

Xylitol, starch and xanthan gum were fully mixed and transferred to a dropping machine, to which phenobarbital was added. The mixture was stirred to homogenize, heated to be molten using a water-bath at temperature of 90°C. Dropping the melted mixture at temperature of 85°C into coolant of liquid paraffin at temperature of 10°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 4.53min without baffle, which complied with the requirements of the Chinese Pharmacopoeia.

### Example 44:

The corresponding extract was prepared according to the preparation method of huoxiangzhengqi liquid described in Chinese Pharmacopoeia.

Said extract 12g, lactitol 30g, starch 20g, arabic gum 5g.

Lactitol, starch and arabic gum were fully mixed and transferred to a dropping machine, the extract, to which the extract, volatile oil of *Herba Pogostemonis* and perilla leaf oil were added. The mixture was stirred to homogenize, heated to be molten using a water-bath at temperature of 80°C. Dropping the melted mixture at temperature of 65°C into coolant of liquid paraffin at temperature of 4°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.79min without baffle, which complied with the requirements of the Chinese Pharmacopoeia.

### Example 45:

Griseofulvin 5g, fructose 20g, sorbitol 10g, sodium alginate 20g.

Fructose, sorbitol and sodium alginate were fully mixed and transferred to a dropping machine, to which griseofulvin was added, stirred to homogenize the mixture, heated to be molten using a water-bath at temperature of 89°C. Dropping the melted mixture at temperature of 82°C into coolant of liquid paraffin at temperature of 3°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.58min without baffle, which complied with the requirements of the Chinese Pharmacopoeia.

### Example 46:

Chloramphenicol 6g, lactitol 30g, carrageen gum 3g, dextrin 5g.

Lactitol, carrageen gum and dextrin were fully mixed and transferred to a dropping machine, to which chloramphenicol was added. The mixture was stirred to homogenize, heated to be molten using a water-bath at temperature of 95°C. Dropping the melted mixture at temperature of 90°C into coolant of liquid paraffin at temperature of 5°C at the speed of 40 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 4.13min without baffle, which complied with the requirements of the Chinese Pharmacopoeia.

### Example 47:

Compound norgestrel 6g, lactitol 26g, isomaltitol 10g, tragacanth 5g.

Lactitol, isomaltitol and tragacanth were fully mixed and transferred to a dropping machine, to which compound norgestrel was added. The mixture was stirred to homogenize the mixture, heated to be molten using a water-bath at temperature of 89°C. Dropping the melted mixture at temperature of 80°C into coolant of liquid paraffin at temperature of 4°C at the speed of 35 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 5.67min without baffle, which complied with the requirements of the Chinese Pharmacopoeia.

### Example 48:

Spironolactone 5g, lactitol 35g, isomaltitol 25g, starch 8g.

Lactitol, isomaltitol and starch were fully mixed and transferred to a dropping machine, spironolactone was dissolved with 95% ethanol and added to the above mixture. The mixture was stirred to homogenize, heated to be molten using a water-bath at temperature of 85°C. Dropping the melted mixture at temperature of 80°C into coolant of liquid paraffin at temperature of 4°C at the speed of 40 pellets/min. After shaping, the liquid paraffin on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 4.55min without baffle, which complied with the requirements of the Chinese Pharmacopoeia.

### Example 49:

*Radix Ginseng*, *Radix Ophiopogonis* and *Fructus Schisandrae* were mixed with the weight ratio of 1:2:1, and the mixture was prepared to obtain the extract.

Said extract 15g, xylitol 35g, hydroxypropyl methyl cellulose 12g, starch 6g.

Xylitol was added into the extract, fully stirred, and heating to be molten using a water-bath at temperature of 90°C. Then the mixture was transferred to a dropping machine. Hydroxypropyl methyl cellulose and starch were mixed, heated and stirred to homogenize, and transferred to a dropping machine. Dropping the melted mixture at temperature of 80°C into coolant of dimethyl silicone oil at temperature of 10°C at the speed of 40 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 2.98min without baffle, which complied with the requirements of the Chinese Pharmacopoeia.

### Example 50:

*Rhizoma Coptidis, Cortex Phellodendri, Fructus Gardeniae,* and *Radix Scutellariae* were mixed with the weight ratio of 1.6:1.1:1.1:2.2, and the mixture was extracted with water, precipitated with alcohol, then concentrated to obtain the extract.

Said extract 12g, sucrose ester 30g, polyoxyethylene monostearate 12g, cross-linked sodium carboxyl methyl cellulose 18g.

Sucrose ester was added into the extract, fully stirred, and heated to be molten using a water-bath at temperature of 90°C.Polyoxyethylene monostearate was solely molten, and mixed with the above mixture, and then transferred the mixture to a dropping machine; to which cross-linked sodium carboxyl methyl cellulose was added. The melted mixture at temperature of 85°C was dropped into coolant of dimethyl silicone oil at temperature of 15°C at the speed of 30 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.76min without baffle, which complied with the requirements of the Chinese Pharmacopoeia.

### Example 51:

Extract of leech (*Whitmania pigra Whitman*) 20g, sucrose ester 40g, polyoxyethylene monostearate 20g.

Sucrose ester was added into the extract, fully stirred, and heated to be molten using a water-bath at temperature of 85°C. Polyoxyethylene monostearate was solely molten, and mixed with the above mixture, and then transferred the mixture to a dropping machine. The melted mixture at temperature of 80°C was dropped into coolant of dimethyl silicone oil at temperature of 18°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 4.10min without baffle, which complied with the requirements of the Chinese Pharmacopoeia.

### Example 52:

*Radix Bupleuri* was extracted with hot water, precipitated using alcohol, and then concentrated to obtain extract.

Said extract 18g, sucrose ester 20g, glycerin monostearate 10g.

Sucrose ester was added into the extract, fully stirred, and heated to be molten using a water-bath at temperature of 85°C. Glycerin monostearate was solely molten, and mixed with the above mixture, and then transferred the mixture to a dropping machine. The melted mixture at temperature of 75°C was dropped into coolant of dimethyl silicone oil at temperature of 24°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.25min without baffle, which complied with the requirements of the Chinese Pharmacopoeia.

### Example 53:

The volatile oil of *Radix Bupleuri* was obtained by method of supercritical extraction, the residue was extracted to obtain saikosaponin as the effective fractions, and then the extract was obtained.

Said extract 20g, sucrose ester 20g, glycerin monostearate 14g.

Sucrose ester was added into the extract, fully stirred, and heated to be molten using a water-bath at temperature of 85°C. Glycerin monostearate was solely molten, mixed with the above mixture, and then transferred the mixture to a dropping machine. The melted mixture at temperature of 75°C was dropped into coolant of dimethyl silicone oil at temperature of 28°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.25min without baffle, which complied with the requirements of the Chinese Pharmacopoeia.

### Example 54:

Oil of *Blumea balsamifera (L.) DC.,* 14g; borneol, 1g; sucrose ester, 40g; polyoxyethylene monostearate, 21 g.

Sucrose ester and borneol were added into oil of *Blumea balsamifera (L.)* DC., fully stirred, and heated to be molten using a water-bath at temperature of 85°C. Polyoxyethylene monostearate was solely molten, mixed with the above mixture, and then transferred the mixture to a dropping machine. The melted mixture at temperature of 80°C was dropped into coolant of dimethyl silicone oil at temperature of 26°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.43min without baffle, which complied with the requirements of the Chinese Pharmacopoeia.

### Example 55:

Extract of *Radix Salviae Miltiorrhizae* and *Radix Notoginseng* (Chinese Patent No. CN 1348815A): 12g; borneol 1.2g; sucrose ester 28g; polyoxyethylene monostearate 14g.

Sucrose ester and borneol were added into the extract, fully stirred, and heated to be molten using a water-bath at temperature of 85°C. Polyoxyethylene monostearate was solely molten, mixed with the above mixture, and then transferred the mixture to a dropping machine. The melted mixture at temperature of 64°C was dropped into coolant of dimethyl silicone oil at temperature of 30°C at the speed of 40 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.63min without baffle, which complied with the requirements of the Chinese Pharmacopoeia.

### Example 56:

Extract of *Radix Puerariae* (the content of pueraria flavones is more than 80%),15g; sucrose ester, 30g; polyoxyethylene monostearate, 9g; cross-linked sodium carboxyl methyl cellulose, 15g.

Sucrose ester was added into the extract, fully stirred, and heated to be molten using a water-bath at temperature of 90°C. Polyoxyethylene monostearate was solely molten, mixed with the above mixture, and then transferred the mixture to a dropping machine, to which cross-linked sodium carboxyl methyl cellulose was added. The melted mixture at temperature of 85°C was dropped into coolant of dimethyl silicone oil at temperature of 23°C at the speed of 40 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.64min without baffle, which complied with the requirements of the Chinese Pharmacopoeia.

### Example 57:

Extract of *Radix Puerariae*(the content of pueraria flavones is more than 40%, and the content of puerarin is more than 28%), 12g; sucrose ester, 30g; polyoxyethylene monostearate, 6g; cross-linked sodium carboxyl methyl cellulose, 18g.

Sucrose ester was added into the extract, fully stirred, and heated to be molten using a water-bath at temperature of 90°C. Polyoxyethylene monostearate was solely molten, mixed with the above mixture, and then transferred the mixture to a dropping machine, to which cross-linked sodium carboxyl methyl cellulose was added. The melted mixture at temperature of 85°C was dropped into coolant of dimethyl silicone oil at temperature of 19°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination, Determination results of disintegration time by the method described in Chinese Pharmacopoeia (2000) revealed that the drop pills passed the wire mesh completely within the mean time of 3,15min without baffle, which complied with the requirements of the Chinese Pharmacopoeia.

### Example 58:

*Oleum Rhododendri Daurici* 12g, borneol 1.2g, sucrose ester 28g. polyoxyethylene monostearate 25.2g.

Sucrose ester was added into *Oleum Rhododendri Daurici,* fully stirred, and heated to be molten using a water-bath at temperature of 85°C. Polyoxyethylene monostearate was solely molten, mixed with the above mixture, and then transferred the mixture to a dropping machine. The melted mixture at temperature of 70°C was dropped into coolant of dimethyl silicone oil at temperature of 25°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.63min without baffle, which complied with the requirements of the Chinese Pharmacopoeia.

### Example 59:

*Oleum Viticis Negundo* 12g, sucrose ester 35g, polyoxyethylene monostearate 12g.

Sucrose ester was added into *Oleum Viticis Negundo,* fully stirred, and heated to be molten using a water-bath at temperature of 80°C. Polyoxyethylene monostearate was solely molten, mixed with the above mixture, and then transferred the mixture to a dropping machine. The melted mixture at temperature of 65°C was dropped into coolant of dimethyl silicone oil at temperature of 22°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.55min without baffle, which complied with the requirements of the Chinese Pharmacopoeia.

### Example 60:

Extract of *Radix Salviae Miltiorrhizae* and *Radix Notoginseng* (Chinese Patent No. CN 1348815A): 22g; borneol 1.5g; sucrose ester 40g; polyoxyethylene monostearate 15g.

Sucrose ester was added into the extract and borneol, fully stirred, and heated to be molten using a water-bath at temperature of 85°C. Polyoxyethylene monostearate was solely molten, mixed with the above mixture, and then transferred the mixture to a dropping machine. The melted mixture at temperature of 64°C was dropped into coolant of dimethyl silicone oil at temperature of 18°C at the speed of 40 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 4.25min without baffle, which complied with the requirements of the Chinese Pharmacopoeia.

### Example 61:

Extracts derived from *Flos Lonicerae, Radix Scutellariae, Fructus Forsythiae* with the weight ratio of 1:1:2.

Said extracts 20g, sucrose ester 35g, polyoxyethylene monostearate 15g.

Sucrose ester was added into the extract, fully stirred, and heated to be molten using a water-bath at temperature of 95°C. Polyoxyethylene monostearate was solely molten, and mixed with the above mixture, and then transferred the mixture to a dropping machine. The melted mixture at temperature of 64°C was dropped into coolant of dimethyl silicone oil at temperature of 10°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.68min without baffle, which complied with the requirements of the Chinese Pharmacopoeia.

### Example 62:

Styrallyl ester 22g, sucrose ester 30g, polyoxyethylene monostearate 20g, cross-linked sodium carboxyl methyl cellulose 2g, silicon dioxide (food grade) 2g.

Sucrose ester was added into styrallyl ester, fully stirred, and heated to be molten using a water-bath at temperature of 85°C. Polyoxyethylene monostearate was solely molten, mixed with the above mixture, and then transferred the mixture to a dropping machine, to which cross-linked sodium carboxyl methyl cellulose and silicon dioxide (food grade) were added. The melted mixture at temperature of 75°C was dropped into coolant of dimethyl silicone oil at temperature of 30°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.10min without baffle, which complied with the requirements of the Chinese Pharmacopoeia.

### Example 63:

The volatile oil of *Rhizoma Chuanxiong* was obtained by method of supercritical extraction, the residue was extracted with the low concentration ethanol, and then concentrated to obtain the extract.

The extract 22g, sucrose ester 30g, polyoxyethylene monostearate 22g, cross-linked sodium carboxyl methyl cellulose 4g, silicon dioxide (food grade) 4g.

Sucrose ester was added into the extract, fully stirred, and heated to be molten using a water-bath at temperature of 85°C. Polyoxyethylene monostearate was solely molten, mixed with the above mixture, and then transferred the mixture to a dropping machine, to which cross-linked sodium carboxyl methyl cellulose and silicon dioxide (food grade) were added. The melted mixture at temperature of 65°C was dropped into coolant of dimethyl silicone oil at temperature of 20°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.22min without baffle, which complied with the requirements of the Chinese Pharmacopoeia.

### Example 64:

The extract of *Erigeron brevsicapus (Vant) Hand-Mazz, Folium Ginkgo, Radix Salviae Miltorrhizae* and natural borneol was prepared according to the method of yinzhanxinmai drop pills in National Specification Compilation of Traditional Chinese Patent Medicines Preparations, Part of Internal medicine Heart.

Said extract 22g, sucrose ester 30g, polyoxyethylene monostearate 14g, cross-linked sodium carboxyl methyl cellulose 0.2g, silicon dioxide (food grade) 0.2g.

Sucrose ester was added into the extract, fully stirred, and heated to be molten using a water-bath at temperature of 90°C. Polyoxyethylene monostearate was solely molten, mixed with the above mixture, and then transferred the mixture to a dropping machine, to which cross-linked sodium carboxyl methyl cellulose and silicon dioxide (food grade) were added. The melted mixture at temperature of 75°C was dropped into coolant of dimethyl silicone oil at temperature of 21°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 4.16min without baffle, which complied with the requirements of the Chinese Pharmacopoeia.

### Example 65:

The ethyl ether extract of *Rhizoma Chuanxiong,* 33g; the ethyl ether extract of *Radix Angelicae Sinensis,* 31 g; sucrose ester, 30g; polyoxyethylene monostearate, 27g; cross-linked sodium carboxyl methyl cellulose, 24g;

Sucrose ester was added into the extract, fully stirred, and heated to be molten using a water-bath at temperature of 85°C Polyoxyethylene monostearate was solely molten, mixed with the above mixture, and then transferred the mixture to a dropping machine, to which cross-linked sodium carboxyl methyl cellulose was added. The melted mixture at temperature of 70°C was dropped into coolant of dimethyl silicone oil at temperature of 10°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 3.35min without baffle, which complied with the requirements of the Chinese Pharmacopoeia.

### Example 66:

*Sonqi tofal Saponins std.* 12g, sucrose ester 30g, polyoxyethylene monostearate 6g, cross-linked sodium carboxyl methyl cellulose 21g.

Sucrose ester was added into the extract, fully stirred, and heated to be molten using a water-bath at temperature of 90°C. Polyoxyethylene monostearate was solely molten, mixed with the above mixture, and then transferred the mixture to a dropping machine, to which cross-linked sodium carboxyl methyl cellulose was added. The melted mixture at temperature of 85°C was dropped into coolant of dimethyl silicone oil at temperature of 10°C at the speed of 35 pellets/min. After shaping, the dimethyl silicone oil on the surface of the drop pills was absorbed using absorbing paper, and then the drop pills were obtained by drying at a low temperature. The results indicated that the produced drop pills were sphere with even size, uniform color and without conglutination. Determination results of disintegration time by the method described in Chinese Pharmacopoeia (2000) revealed that the drop pills passed the wire mesh completely within the mean time of 4.65min without baffle, which complied with the requirements of the Chinese Pharmacopoeia.

In order to better understand the present invention, hereinafter the advantages of the present invention are further explained by way of some experiments, such as disintegration time, weight variation, rigidity, viscidity of the drop pills prepared by the matrix adjuvants of Example 10.

### Experimental example 1:

### Comparative experimental example of disintegration time and weight variation

In comparing the drop pills prepared by the matrix adjuvant of this invention with those prepared by polyethylene glycol respectively, the disintegration time was measured to determine whether the former drop pills have satisfactory release effect, and the weight variation and other indexes were measured to determine whether the preparation process is mature and adaptable to the industrial applicability.

### 1. Samples:

The drop pills prepared with the extract of Chinese medicines disclosed in Chinese Patent No. CN 1348815A as active ingredient and the novel matrix adjuvant of this invention as matrix adjuvant (hereinafter labeled as "new"); the drop pills prepared with the extract of Chinese medicines disclosed in Chinese Patent No. CN 1348815A as active ingredient and polyethylene glycol as matrix adjuvant (hereinafter labeled as "known").

### 2. Method and results:

Disintegration limit: measured in the method of the corresponding item in Chinese Pharmacopoeia; weight variation: measured in the method of the corresponding item in Chinese Pharmacopoeia. Results are shown in Table 1.

**Table 1: Comparison of disintegration limit and weight variation of three batches of the drop pills between those prepared by the novel matrix adjuvant (labeled as "new") with those prepared by polyethylene glycol as the main adjuvant (labeled as "known")**

| | | | 0 month | 1^{st} month | 2^{nd} month | 3^{rd} month | 6^{th} month | 12^{th} month | 18^{th} month |
|---|---|---|---|---|---|---|---|---|---|
| Criterion | | | Results | | | | | | |
| 1^{a} batch | Weight variation (±15%) | | within ± 10% | within ±10% | within ±10% | within ±10% | Within ±10% | within ±10% | Within ± 1096 |
| | Disintegration time | (new) | 2'05" | 2'09" | 2'16" | 2'15" | 2'16" | 2'20" | 2'23" |
| | | (known) | 5'11" | 5'06" | 5'15" | 5'19" | 5'26" | 5'16" | 5'35" |
| 2^{nd} batch | Weight variation (±15%) | | within ±10% | within ±10% | within ±10% | within ±10% | Within ±10% | within ± 10% | within ±10% |
| | Disintegration time | (new) | 1'57" | 1'59" | 1'56" | 2'04" | 2'09" | 2'10" | 2'08" |
| | | (known) | 5'14" | 5'18" | 5'21" | 5'19" | 5'26" | 5'34" | 5'32" |
| 3^{rd} batch | Weight variation (±15%) | | within ±10% | within ±10% | within ±10% | within ±10% | Within ±10% | within ±10% | within ±10% |
| | Disintegration time | (new) | 2'12" | 2'09" | 2'15" | 2'13" | 2'17" | 2'20" | 2'25" |
| | | (known) | 5'10" | 5'17" | 5'21" | 5'23" | 5'26" | 5'30" | 5'37" |

The experiment data indicated that the disintegration limit of drop pills prepared by the novel matrix adjuvant is less than those prepared by polyethylene glycol as the main adjuvant and that the weight variation of new or known drop pills are controlled within the required range in Chinese Pharmacopoeia. The experiment data also indicated the disintegration speed of the drop pills prepared by the novel matrix adjuvant is more quick and more favorable for making active ingredient take effect in a time as short as possible. The weight variation of new or known drop pills are controlled within the required range in Chinese Pharmacopoeia, the variation between them is not notable in statistics. Therefore, said matrix adjuvant can replace the current chemically synthesized adjuvants for industrial production.

### Experimental example 2:

Comparative experimental example of rigidity and viscidity of the drop pills between those prepared by the matrix adjuvants of the present invention and those prepared by polyethylene glycol as the main adjuvant

### 1. Samples:

The drop pills (new) prepared with the extract of Chinese medicines disclosed in Chinese Patent No. CN 1348815A as active ingredient and the novel matrix adjuvants of this invention as matrix adjuvants (hereinafter labeled as "new"); the drop pills(known) prepared with the extract of Chinese medicines disclosed in Chinese Patent No. CN 1348815A as active ingredient and polyethylene glycol as matrix adjuvants (hereinafter labeled as "known").

### 2. Method and results:

Three batches of drop pills was taken, placed into the porcelain bottles respectively and sealed tightly with the bottle stoppers; the sealed bottles were placed into a desiccator with saturated NaCl solution (humidity 75%) in its bottom, and then the desiccator was put into a drying cabinet at constant temperature of 40°C. Samples were collected at regular intervals to examine the rigidity and viscidity of drop pills. Results are shown in Table 2.

**Table 2: Comparison of characters of three batches of the drop pills between those prepared by the novel matrix adjuvant (labeled as "new") with those prepared by polyethylene glycol as the adjuvant (labeled as "known")**

| | | 0 month | 1^{st} month | 2^{nd} month | 3^{rd} month | 6^{th} month | 12^{th} month | 18^{th} month |
|---|---|---|---|---|---|---|---|---|
| Criterion | | Results | | | | | | |
| 1^{st} batch | viscidity | *(known) | *(known) | *(known) | *(known) | **(known) | **(known) | ***(known) |
| | | *(new) | *(new) | *(new) | *(new) | *(new) | **(new) | **(new) |
| | rigidity | A(known) | A(kwown) | A(known) | A(known) | B(known) | C(known) | C(known) |
| | | A(new) | A(new) | A(new) | A(new) | A(new) | A(new) | C(new) |
| 2^{nd} batch | viscidity | *(know) | *(known) | *(known) | *(known) | *(known) | **(known) | **(known) |
| | | *(new) | *(new) | *(new) | *(new) | *(new) | *(new) | **(new) |
| | rigidity | A(known) | A(known) | A(known) | A(known) | B(known) | C(known) | C(known) |
| | | A(new) | A(new) | A(new) | A(new) | A(new) | A(new) | C(new) |
| 3^{rd} batch | viscidity | *(known) | *(known) | *(known) | *(known) | **(known) | **(known) | ***(known) |
| | | *(new) | *(new) | *(new) | *(new) | *(new) | *(new) | **(new) |
| | rigidity | A(known) | A(known) | A(known) | A(known) | A(known) | C(known) | C(known) |
| | | A(new) | A(new) | A(new) | A(new) | A(new) | A(new) | C(new) |

| | | | | | | | | |
|---|---|---|---|---|---|---|---|---|
| Note: *= not sticky; **=a little sticky; ***= sticky; A=Hard; B=less hard than normal; C=much less hard than normal. | | | | | | | | |

The experiment data indicated that, compared with those prepared by polyethylene glycol, the variation of rigidity of drop pill prepared by the novel matrix adjuvant is similar or a little bigger, the viscidity is similar. The experiment data also indicated the rigidity and viscidity variation between new and known drop pills are similar. Therefore, said matrix adjuvant can replace the current chemically synthesized adjuvants for industrial production.

### Industrial applicability

In addition to the advantages of the conventional drop pills, such as easy preparation, stable quality, solidifying the liquid active ingredient, convenient administration, and high and quick efficacy, the greatest advantage of the drop pills prepared in the present invention lies in the following: the matrix adjuvants used in the present invention are derived from the natural plants, or mainly comprises the matrix adjuvant derived from the natural plants. The matrix adjuvants derived from the natural plants are not only pharmaceutically acceptable, but are commonly used as additives in the food industry. Since said matrix adjuvant is not only absolutely safe without any toxic and side effects, but also very cheap and accessible, it has great value for application and popularization, thus laying a solid foundation for the internationalization of said drop pills.

Meanwhile, the rigidity and viscidity variation of the drop pill prepared by the novel matrix adjuvant of the present invention is similar to that of the drop pill prepared by polyethylene glycol as matrix adjuvant, which indicated that the natural matrix adjuvant can replace the current chemically synthesized adjuvants for industrial production.

## Claims

1. A drop pill comprising the pharmaceutical active ingredient and at least one of the pharmaceutically acceptable matrix adjuvants selected from the group consisting of D-ribose, fructose, xylose, trehalose, raffinose, maltose, gelose, D-ribonic acid-γ-lactone, erythritol, arabitol, isomaltitol, lactitol and malic acid, and the above-mentioned compounds containing crystal water, wherein the drop pill is prepared by dripping, namely, a solid or liquid drug is dissolved, suspended or emulsified into a matrix, then this mixture is dropped into a matrix-insoluble coolant, the temperature of the coolant being -20 to 30 °C.

2. The drop pill according to claim 1, wherein said pharmaceutical active ingredient is an extract of crude drug.

3. The drop pill according to claim 1, wherein said pharmaceutical active ingredient is a chemically synthesized drug, antibiotic or biochemical drug.

4. The drop pill according to any one of claims 1-3, wherein said drop pill further comprises at least one of plastifying components selected from the group consisting of the following : starch and their derivatives, cellulose and their derivatives, arabic gum, dextran, chitin, sesbania gum, carrageen gum, Indian gum, danish agar, tragacanth gum, carrageenin, tamarind gum, pectin, xanthan gum, alginic acid and the salts thereof, dextrin, cyclodextrin, agar, lactose; polyvinylpyrrolidone, crosslinked polyvinylpyrrolodione, carbomer, polyvinyl alcohol, acrylic acid resin, poloxamer, silicon dioxide, glutin, glycerin monostearate, and polyoxyethylene monostearate.

5. The drop pill according to claim 4, wherein said plastifying components is one or more substances selected from the group consisting of the following: pregelatinized starch, carboxymethyl starch, methyl cellulose, sodium carboxymethyl cellulose, hydroxypropyl methyl cellulose, arabic gum, alginic acid, dextrin, cyclodextrin, agar, lactose, glycerin monostearate, polyoxyethylene monostearate, cross-linked sodium carboxylmethyl cellulose, and silicon dioxide.

6. The drop pill according to claim 1, wherein the weight ratio of the matrix adjuvant to the pharmaceutical active ingredient is in the range of 1:0.1 ∼ 1:1.

7. The drop pill according to claim 1, wherein the weight ratio of matrix adjuvant to the pharmaceutical active ingredient is in the range of 1:0.1∼1:0.6.

## Patentansprüche

1. Tropfenpille umfassend den pharmazeutisch aktiven Inhaltsstoff und mindestens einen der pharmazeutisch akzeptablen Matrixhilfsstoffe ausgewählt aus der Gruppe bestehend aus D-Ribose, Fructose, Xylose, Trehalose, Raffinose, Maltose, Gelose, D-Ribonsäure-γ-lacton, Erythritol, Arabitol, Isomaltitol, Lactitol und Äpfelsäure und die oben genannten Verbindungen enthaltend Kristallwasser, wobei die Tropfenpille durch Tropfen zubereitet wird, und zwar wird ein festes oder flüssiges Medikament in einer Matrix gelöst, suspendiert oder emulgiert, dann wird diese Mischung in ein Matrix-unlösliches Kühlmittel getropft, wobei die Temperatur des Kühlmittels -20 bis 30 °C beträgt.

2. Tropfenpille gemäß Anspruch 1, wobei der genannte pharmazeutisch aktive Inhaltsstoff ein Extrakt von einem Roh-Medikament ist.

3. Tropfenpille gemäß Anspruch 1, wobei der genannte pharmazeutisch aktive Inhaltsstoff ein chemisch synthetisiertes Medikament, ein antibiotisches oder biochemisches Medikament ist.

4. Tropfenpille gemäß einem der Ansprüche 1-3, wobei die genannte Tropfenpille weiterhin mindestens eine Plastifizierkomponente ausgewählt aus der Gruppe bestehend aus den Folgenden umfasst: Stärke und ihre Derivate, Cellulose und ihre Derivate, Arabicum-Gummi, Dextran, Chitin, Sesbania-Gummi, Carrageen-Gummi, Indisches-Gummi, Dänischer Agar, Traganth-Gummi, Carrageene, Tamarinden-Gummi, Pektine, Xanthan-Gummi, Alginsäure und die Salze davon, Dextrin, Cyclodextrin, Agar, Lactose; Polyvinylpyrrolidon, quervernetztes Polyvinylpyrrolidon, Carbomer, Polyvinylalkohol, Acrylsäureharz, Poloxamer, Siliciumdioxid, Glutin, Glycerinmonostearat, und Polyoxyethylenmonostearat.

5. Tropfenpille gemäß Anspruch 4, wobei die genannten Plastifizierkomponenten eine oder mehrere Substanzen ausgewählt aus der Gruppe bestehend aus den Folgenden ist: Vorgelierte Stärke, Carboxymethylstärke, Methylcellulose, Natriumcarboxymethylcellulose, Hydroxypropylmethylcellulose, Arabicum-Gummi, Alginsäure, Dextrin, Cyclodextrin, Agar, Lactose, Glycerinmonostearat, Polyoxyethylenmonostearat, quervernetze Natriumcarboxylmethylcellulose, und Siliciumdioxid.

6. Tropfenpille gemäß Anspruch 1, wobei der Gewichtsanteil des Matrixhilfsstoffes zu dem pharmazeutisch aktiven Inhaltsstoff in dem Bereich von 1:0,1∼ 1:1 ist.

7. Tropfenpille gemäß Anspruch 1, wobei der Gewichtsanteil des Matrixhilfsstoffes zu dem pharmazeutisch aktiven Inhaltsstoff in dem Bereich von 1:0,1∼ 1:0,6 ist.

## Revendications

1. Comprimé lingual comprenant le principe actif pharmaceutique et au moins un des adjuvants matriciels pharmaceutiquement acceptables choisis dans le groupe constitué par le D-ribose, le fructose, le xylose, le tréhalose, le raffinose, le maltose, la gélose, l'acide D-ribonique-γ-lactone, l'érythritol, l'arabitol, l'isomaltitol, le lactitol et l'acide malique, et les composés mentionnés ci-dessus contenant de l'eau cristalline, le composé lingual étant préparé par stillation, c'est-à-dire qu'un médicament solide ou liquide est dissous, suspendu ou émulsifié dans une matrice, puis ce mélange est déversé dans un liquide de refroidissement insoluble dans la matrice, la température du liquide de refroidissement étant de -20 à 30 °C.

2. Comprimé lingual selon la revendication 1, dans lequel ledit principe actif pharmaceutique est un extrait de médicament brut.

3. Comprimé lingual selon la revendication 1, dans lequel ledit principe actif pharmaceutique est un médicament chimiquement synthétisé, un antibiotique ou un médicament biochimique.

4. Comprimé lingual selon l'une quelconque des revendications 1 à 3, dans lequel ledit comprimé lingual comprend en outre au moins un des composants plastifiants choisis dans le groupe constitué par les composés suivants : un amidon et ses dérivés, une cellulose et ses dérivés, la gomme arabique, le dextrane, la chitine, la gomme Sesbania, la gomme de carraghénane, la gomme ghatti, la furcellarane, la gomme adragante, la carraghénine, la gomme de taramin, la pectine, la gomme de xanthane, l'acide alginique et ses sels, la dextrine, la cyclodextrine, la gélose, le lactose ; la polyvinylpyrrolidone, la polyvinylpyrrolodione réticulée, un carbomère, un polyalcool de vinyle, une résine d'acide acrylique, un poloxamère, le dioxyde de silicium, la glutine, le monostéarate de glycérine et le monostéarate de polyoxyéthylène.

5. Comprimé lingual selon la revendication 4, dans lequel ledit composant plastifiant est une ou plusieurs substances choisies dans le groupe constitué par les composés suivants : un amidon prégélatinisé, le carboxyméthylamidon, la méthylcellulose, la carboxyméthylcellulose sodique, l'hydroxypropylméthylcellulose, la gomme arabique, l'acide alginique, la dextrine, la cyclodextrine, la gélose, le lactose, le monostéarate de glycérine, le monostéarate de polyoxyéthylène, la carboxyméthylcellulose sodique réticulée et le dioxyde de silicium.

6. Comprimé lingual selon la revendication 1, dans lequel le rapport pondéral de l'adjuvant matriciel au principe actif pharmaceutique est compris dans la plage allant de 1:0,1 à environ 1:1.

7. Comprimé lingual selon la revendication 1, dans lequel le rapport pondéral de l'adjuvant matriciel au principe actif pharmaceutique est compris dans la plage allant de 1:0,1 à environ 1:0,6.
